**Europäisches Patentamt**

**European Patent Office**

**Office européen des brevets**

(19)

(11) Numéro de publication: **0 343 119**
**A2**

(12)

# DEMANDE DE BREVET EUROPÉEN

(21) Numéro de dépôt: **89830196.5**

(22) Date de dépôt: **09.05.89**

(51) Int. Cl.⁴: **A 61 M 1/00**

(30) Priorité: **13.05.88 IT 6427488**

(43) Date de publication de la demande:
**23.11.89 Bulletin 89/47**

(84) Etats contractants désignés:
**BE CH DE ES FR GB IT LI LU NL**

(71) Demandeur: **Rotellini, Sergio**
**Via dei Mille 90**
**I-36100 Vicenza (IT)**

(72) Inventeur: **Rotellini, Sergio**
**Via dei Mille 90**
**I-36100 Vicenza (IT)**

Le titre de l'invention a été modifié (Directives relatives à l'examen pratiqué à l'OEB, A-III, 7.3)

(54) **Appareil de drainage post-opératoire par dépression.**

(57) L'appareil se compose:
- d'un tuyau de drainage (2a), de la longueur de 50 centimètres,
à enfoncer dens la blessure chirurgicale:
- d'un raccord à deux voies (2b):
- d'un tuyau de raccord de 100 centimètres, branché à
un"bouchon technique" à deux valves:
- d'un soufflet autoaspirant.

En laissant "in situ" le tuyau drainant perforé, la seule
compression du soufflet remet en action l'appareil pour le
drainage du liquide physiologique qui se trouve dans la
blessure.

Il n'est jamais nécessaire de disjoindre les éléments qui
pourraint infecter la blessure chirurgicale.

L'action drainante se produit pourtant d'une façon correcte,
precise, simple, et rigoureusement aseptique.

Le temps de l'action drainante est fixée par la Chirurgien.

EP 0 343 119 A2

Bundesdruckerei Berlin

## Description

### Appareil post-opératoire, autoaspirant, à double valve, antireflux, apte à drainer liquides

Il s'agit d'un appareil pour drainer les liquides physiologiques qui comprend: un tuyau de drainage (2a) de la longueur de 50 centimètres, perforé à une extremité de 15 centimètres; un raccord à deux voies (2b) qui relie le premier tuyau (2a) à un deuxième tuyau branché à un "bouchon technique" à deux valves (4) (5), vissé au soufflet autoaspirant.

Cet appareil, utilisé dans les hôpitaux, permet, après une intervention chirurgicale, le drainage de liquides physiologiques de la blessure pour permettre une rapide cicatrisation.

On connaît déjà des appareils tels qu'on vient de décrire au titre.

L'action de répétition du drainage, actuellement, demande le travail du personnel qualifié, tandis que pour une action tout à fait aseptique le manque de connexion des éléments des appareils employés actuellement, dépourvus de valve antireflux, mettent en évidence la complexité et la manque de sûreté aseptique.

Ce brevet d'invention veut éliminer les inconvénients qu'on vient de décrire.

L'invention, d'après les revendications qui la caractérisent, résoud soit la répétition des mouvements nécessaires pour recréer la dépression du soufflet (1) (3) soit le danger d'infecter la blessure chirurgicale. (2c)

Les avantages relevés par cet invention viennent surtout du fait que les éléments qui composent le "bouchon technique" empêchent toute possibilité de remontée du liquide physiologique non stérile du soufflet vers la blessure.

De plus la semplicité et la sûreté de l'appareil permettent son utilisation à toute personne car il ne presente jamais possibilité d'infection.

Le brevet d'invention est expliqué dans tous ses détails dans les dessins qui se trouvent à la fin de cette publication.

La figure n° 1 représente, en perspective, un appareil réalisé conformément ce brevet d'invention, où le "bouchon technique" (4)(5) est dessiné à l'extérieur du corps de l'appareil dont il fait partie.

La figure n° 2 représente, sur une différente échelle, une vue de l'appareil entier.

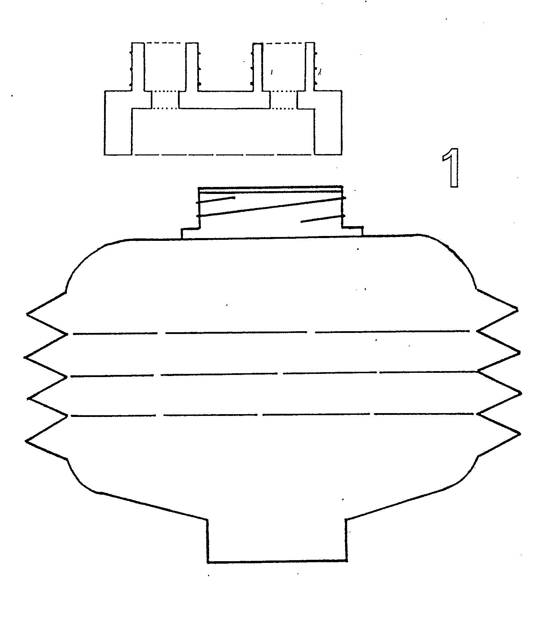

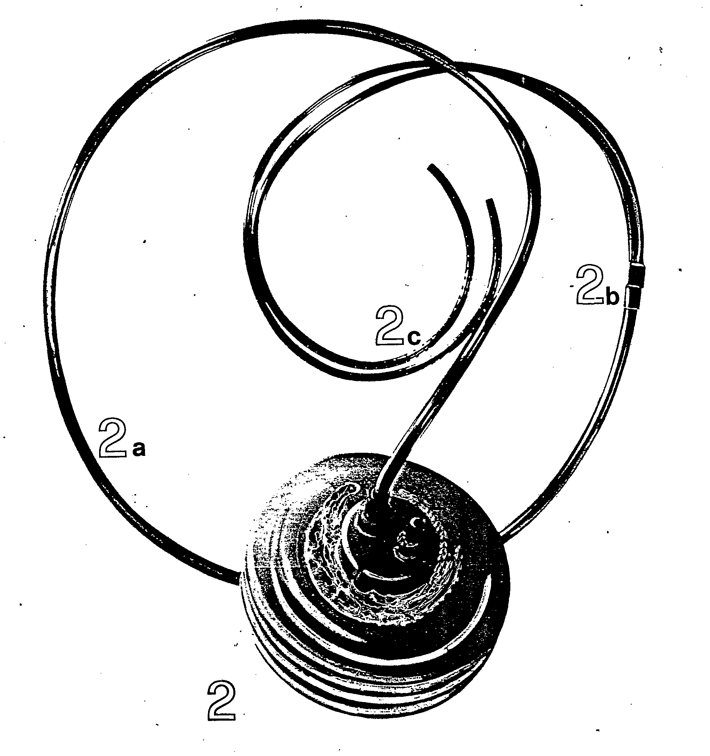

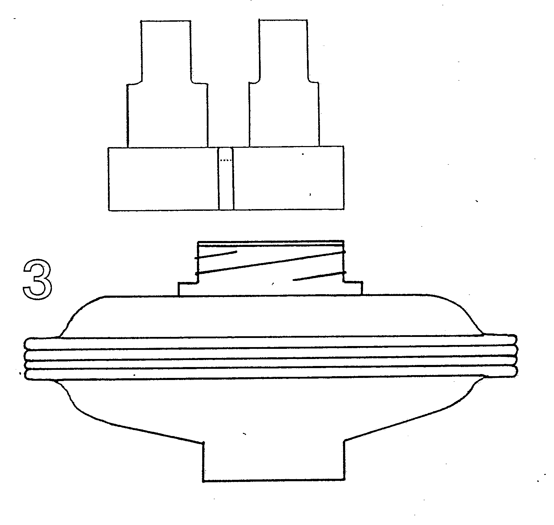

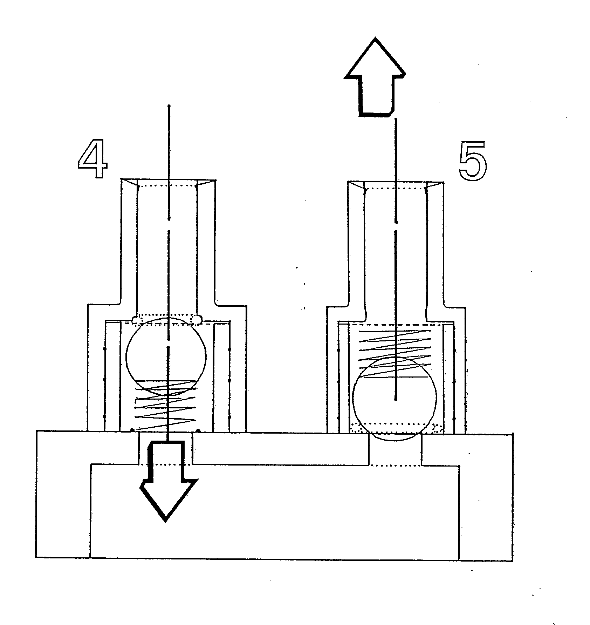

Les figures n° 1 et 3 représentent un appareil drainant, post-opératoire, avec soufflet autoaspirant, à double valve antireflux (4)(5) comprenant le "bouchon technique" qui met en évidence son fonctionnement à la compression et à la dépression: (3)(1) l'air contenu dans le même soufflet sort à travers la valve expirante (5) tandis que par la même compression la valve aspirante (4) se ferme.

Quand le soufflet est relâché, (1)(3) il provoque une dépression, et la valve expirante (5) se ferme, la valve aspirante (4) s'ouvre et le soufflet effectue son action de drainage.

## Revendications

L'action du drainage se développe d'une façon autonome et au cas où il y aurait un mouvement provoquant une compression accidentelle du soufflet, la même compression ferme automatiquement la valve aspirante (4) en empêchant le reflux dans la blessure chirurgicale du liquide qui n'est plus stérile.

Le soufflet a l'avantage d'être remis en action de drainer, tout simplement avec un mouvement de compression (1)(3), jusqu'au moment décidé per le chirurgien.

Pour l'action antireflux de l'appareil, au delà de la caractéristique du "bouchon technique" avec valve antireflux, il y a la forme du même soufflet, (1) dont son hauteur est divisée en 3/3 (et de celle-ci le tiers central se ferme), qui coopère avec l'action antireflux du liquide physiologique.

1

2a

2c

2b

2

3